# EUROPEAN PATENT SPECIFICATION

(11) **EP 2 813 484 B1**
(45) Date of publication and mention of the grant of the patent: **21.12.2016**
(21) Application number: 14171877.5
(22) Date of filing: 11.06.2014
(51) Int. Cl.: C07C 45/28, C07C 45/85, C07C 49/653

(54) **A process for the synthesis of nootkatone**
Verfahren zur Synthese von Nootkaton
Procédé pour la synthèse de la nootkatone

(30) Priority: 11.06.2013 IT MI20130961
(43) Date of publication of application: 17.12.2014
(73) Proprietor: INT Trading S.r.l., 21017 Samarate (VA) (IT)
(72) Inventor: Serra, Stefano, 23807 Merate (LC) (IT)
(74) Representative: Ferreccio, Rinaldo

(56) References cited:
- CN-A- 1 830 936
- US-A1- 2004 014 985
- US-A1- 2008 233 622
- J.A.R. SALVADOR, ET AL.: "Bismuth-catalysed allylic oxidation using t-butyl hydroperoxide", TETRAHEDRON LETTERS, vol. 46, no. 15, 11 April 2005 (2005-04-11), pages 2581-2584, XP004796988, Elsevier Science Publishers, Amsterdam, NL ISSN: 0040-4039, DOI: 10.1016/j.tetlet.2005.02.080
- S.M. SILVESTRE, ET AL.: "Allylic and benzylic oxidation reactions with sodium chlorite", TETRAHEDRON, vol. 63, no. 11, 12 January 2007 (2007-01-12), pages 2439-2445, XP005877827, Elsevier Science Publishers, Amsterdam, NL ISSN: 0040-4020, DOI: 10.1016/j.tet.2007.01.012
- S.F. ARANTES, ET AL.: "The preparation and microbiological hydroxylation of teh sesquiterpenoid nootkatone", JOURNAL OF CHEMICAL RESEARCH (MINIPRINT), no. 3, 1999, pages 801-812, XP008167884, Royal Society of Chemistry, Cambridge, GB ISSN: 0308-2350
- J.A.R. SALVADOR, ET AL.: "The allylic oxidation of unsaturated steroids by tert-butylhydroperoxide using surface functionalised silica supported metal catalysts", GREEN CHEMISTRY, vol. 4, no. 4, 26 June 2002 (2002-06-26), pages 352-356, XP008167883, Royal Socety of Chemistry, Cambridge, GB ISSN: 1463-9262, DOI: 10.1039/b201500p
- H. HIKINO, ET AL.: "Structure and absolute configuration of kusunol", CHEMICAL AND PHARMACEUTICAL BULLETIN, vol. 16, no. 5, 1968, pages 832-838, XP055105579, Pharmaceutical Society of Japan, Tokyo, JP ISSN: 0009-2363, DOI: 10.1248/cpb.16.832
- D.E. CANE, ET AL.: "Preparation of (-)-aristolochene from (+)-valence: absolute configuration of (+)-aristolochene from Aspergillus terreus", TETRAHEDRON LETTERS, vol. 31, no. 14, 1990, pages 1943-1944, XP000650778, Elsevier Science Publishers, Amsterdam, NL ISSN: 0040-4039, DOI: 10.1016/s0040-4039(00)88884-x
- S. TORII, ET AL.: "Functionalisation of trans-decalin. V. A synthesis of (±)-nootkatone and (±)-valencene from 4.beta.,4a.beta.-dimethyl-.delta.6,7-octal in-1-one ethylene acetal.", BULLETIN OF THE CHEMICAL SOCIETY OF JAPAN, vol. 55, no. 3, 1982, pages 887-890, XP055105666, Japan Publications Trading, Tokyo, JP ISSN: 0009-2673, DOI: 10.1246/bcsj.55.887

## Description

### Field of application

The present invention refers to the field of the chemical industry and in particular it concerns a process for the synthesis of nootkatone starting from valencene.

### Prior art

Nootkatone is a sesquiterpene compound that is present in nature in various essential oils such as in the essential oil of vetiver, of Alaskan yellow cedar and especially of grapefruit.

Nootkatone is a compound that is not toxic for humans, it is an authorised additive (flavoring) for food products and is widely used in food products, cosmetic products and pharmaceutical products.

In addition to the uses mentioned above, nootkatone is also used as a repellent/insecticide against mosquitoes and ticks and as a repellent against lice and bugs. Nootkatone is industrially produced by oxidation of valencene, which is in turn a sesquiterpene contained in the essential oil of orange, in particular of Valencia oranges, from which it gets its name.

In the art, different processes are known for preparing nootkatone through oxidation of valencene. Some of these known processes use chrome reagents and they consequently have serious problems in terms of toxicity and of potential environmental pollution.

The Applicant has therefore tried to develop a process that uses less harmful oxidizing agents, which are cost-effective and that can be adapted to industrial production. For such a purpose a known oxidation process seemed particularly interesting, which uses sodium chlorite in combination with tert-butyl hydroperoxide (Tetrahedron, 2007, 63, 2439-2445), which moreover constitutes the object of patent PT 103592 B and does not expressly concern the synthesis of nootkatone.

The application of this process to the synthesis of nootkatone from valencene has however led to unsatisfactory results, since low conversion rates were obtained (50-60%) over quite long time periods (24-48 hours) with product yield, which was isolated after chromatography, of around 30%.

Processes for the oxidation of valencene to nootkatone with tertiary butyl hydroperoxide are known in the presence of bismuth(III) chloride (Tetrahedron Letters, 2005, 46, 2581-2584), manganese(II) acetate (US 2008/0233622 A1) and copper(I) iodide (Journal of Chemical Research (Miniprint), 1999, 801-812). The oxidation is also known using a mixture of sodium hyperchlorite and tertiary butyl hydroperoxide (CN 1 830 936 A).

It was also known to oxidize olefinic compounds with tert-butyl peroxide with a Pd/C 10% catalyst (Organic Letters 2002, 4, 2727-2730). It was then attempted to oxidize valencene with this process and positive results were obtained, obtaining the isolation of nootkatone with around 50% yield. However, such process has the drawback of requiring extreme caution in removing the catalyst in question at the end of the reaction, because possible residual traces of such catalyst could catalyze the degradation of the product during distillation or storage. Moreover, the yield is not completely satisfactory.

The purpose of the present invention was to provide a process for the synthesis of nootkatone from valencene, which could overcome the drawbacks shown by the processes of the prior art and which might allow to obtain better yields than those achieved with such processes, with the use of reagents having reduced hazardousness in terms of toxicity and of pollution as well as being cost-effective, so as to ensure that the process can be adapted to industrial production.

### Summary of the invention

The aforementioned purpose was achieved, according to the invention, by providing a process for the synthesis of nootkatone from valencene, comprising the reaction of valencene with a tertiary alkyl hydroperoxide, in the presence of MnO₂

Preferably, the aforementioned tertiary alkyl hydroperoxide is selected from the group comprising tert-butyl hydroperoxide, tert-amyl hydroperoxide and di-tert-amyl hydroperoxide, advantageously it is tert-butyl hydroperoxide.

The reaction is generally carried out in a non-polar or poorly polar solvent, advantageously in dichloromethane.

The reaction is preferably carried out at a temperature that is lower than or equal to 70°C, advantageously lower than or equal to 40°C and advantageously between -30°C and 40°C.

Preferably, the molar ratio between tertiary alkyl hydroperoxide and valencene varies from 2:1 to 3:1 and is advantageously between 2.2:1 and 2.5.1.

Preferably, the manganese dioxide is used in a molar ratio with respect to the valencene that can vary from 6:1 to 12:1.

Preferably, the process according to the invention comprises the steps of:
a) preparing a suspension of MnO₂ and valencene in a molar ratio from 6:1 to 12:1 in dichloromethane;
b) adding to this suspension tert-butyl hydroperoxide in a molar ratio with respect to the valencene that is variable from 2:1 and 3:1, at a temperature comprised between -30°C and -10°C;
c) maintaining the mixture thus obtained under stirring at a temperature of between -20°C and -10°C for 5-8 hours;
d) gradually raising the temperature to bring the mixture to reflux temperature and keeping it at that temperature for at least 2 hours.

Preferably, in the aforementioned step b), the tert-butyl hydroperoxide is added to the suspension in a molar ratio with respect to valencene that is variable from 2.2:1 to 2.5:1 and in step d) the mixture is maintained at reflux temperature for 2.5-4 hours.

At the end of the reaction the manganese dioxide is separated from the reaction mixture by filtration. The manganese dioxide that is recovered by filtration and washed with dichloromethane or another non-polar solvent can be reused in subsequent reaction cycles.

Raw nootkatone is recovered from the filtrate in the form of an oil, through the elimination of the solvent and subsequent low-pressure distillation of the residue obtained.

By using, as the starting valencene, a product that is normally available on the market with a titre of > 65%, a distillate is obtained comprising nootkatone with a purity that varies from 45 to 60%, because the distillation does not make it possible to eliminate the impurities that were already present in the initial valencene. The distillate, in any case, has a purity that is already sufficient for many of the uses for which nootkatone is intended.

The nootkatone as obtained from the distillation process can in any case be purified by converting it into a crystalline semicarbazone, which is recrystallized and finally reconverted into highly pure nootkatone through acidic hydrolysis.

### Detailed description

The present invention will be further illustrated with reference to an example, provided here below by way of illustration and not of limitation.

### EXAMPLE

50 g (245 mmols) of valencene (Sigma-Aldrich, titre >65%) and 350 mL of dichloromethane were introduced into a one litre three-necked flask, provided with mechanical stirring, dropping funnel and condenser, obtaining a solution. 180 g of MnO₂ (2,07 mols) were then added and the whole mixture was cooled down to about -25°C. At this point, over about 30 minutes, 75 mL of a 70% tert-butyl hydroperoxide solution in water (Luperox^{®}), equal to 582 mmols, were slowly added under vigorous stirring.

Once the addition was finished, the temperature was allowed to rise to - 10°C and the stirring was continued for around 6 hours.

Subsequently, the temperature was allowed to rise and the mixture was refluxed for around 3 hours. A brief evolution of gas was noted, which is an indication of the decomposition of the non-reacted hydroperoxide. The final reaction mixture was then filtered, so as to remove the manganese dioxide, which was washed with dichloromethane and recovered.

The filtered organic phase was concentrated at the rotary evaporator and the residue obtained was distilled under low pressure. A single fraction (40.5 g) of distillate was obtained, consisting of nootkatone with a titre of around 52%, equal to 97 mmols, corresponding to a yield of 56% considering the titre of around 70% of the initial valencene, and an undistillable residue, which still contained small amounts of nootkatone.

The obtained oil was further purified as described in the following.

30 g of the distillate (72 mmols) were dissolved in 250 mL of 60% aqueous ethanol and were treated with 16 g of semicarbazide chloride (143 mmols) and 18 g of sodium acetate (219 mmols). The mixture obtained was refluxed for around 20 minutes, then it was cooled down in a refrigerator for one night. The precipitated nootkatone semicarbazone was separated by filtration (24 g) and then recrystallized from pure ethanol.

The crystalline solid obtained (18.5 g) was suspended in ethyl ether (150 mL) and was subjected to hydrolysis with sulphuric acid diluted to 8% by weight (150 mL), stirring vigorously for around 4 days.

Once the hydrolysis was finished, the organic phase was separated from the aqueous phase, which was re-extracted twice with 60 mL of ethyl ether. The combined organic phases were then washed with a saturated bicarbonate solution and with brine. Through low-pressure concentration, highly pure nootkatone (titre GC-MS > 95%) in an amount equal to 13.1 g (60 mmols) was obtained, with an overall yield of the purification process equal to 83%.

The GC-MS analysis was carried out by means of an HP-6890 gas chromatograph, using, as a detector, a mass spectrometer (5973 mass detector). An HP-5MS column (30 m x 0.25 mm, 0.25 µm firm thickness Hewlett Packard) was used with the following programme: 60°C (1 min) - 6°C/min - 150°C (1 min) - 12°C/min - 280°C (5 min). Helium was used as a carrier with constant flow at 1 ml/min, split 1/30. The retention times of the valencene and of the nootkatone were 18.80 minutes and 23.37 minutes, respectively.

## Claims

1. A process for the synthesis of nootkatone from valencene, comprising the reaction of valencene with a tertiary alkyl hydroperoxide, in the presence of MnO₂

2. The process according to claim 1, wherein said tertiary alkyl hydroperoxide is selected from the group comprising tert-butyl hydroperoxide, tert-amyl hydroperoxide and di-tert-amyl hydroperoxide.

3. The process according to any one of claims 1 and 2, wherein said reaction is carried out in a non-polar or poorly polar solvent, preferably in dichloromethane.

4. The process according to claim 3, wherein said alkyl hydroperoxide is tert-butyl hydroperoxide.

5. The process according to claim 4, wherein said reaction is carried out at a temperature lower than or equal to 70°C.

6. The process according to claim 5, wherein said reaction is carried out in dichloromethane at a temperature lower than or equal to 40°C, preferably at a temperature between -30°C and 40°C.

7. The process according to any one of the preceding claims, wherein the molar ratio between tertiary alkyl hydroperoxide and valencene varies from 2:1 to 3:1 and preferably is between 2.2:1 and 2,5:1.

8. The process according to claim 7, wherein the manganese dioxide is used in a molar ratio with respect to the valencene variable from 6:1 to 12:1.

9. The process according to claim 4, comprising the steps of:
a) preparing a suspension of MnO₂ and valencene in molar ratio from 6:1 to 12:1 in dichloromethane;
b) adding to this suspension tert-butyl hydroperoxide in a molar ratio with respect to the valencene variable from 2:1 to 3:1, at a temperature comprised between -30°C and -10°C;
c) maintaining the mixture thus obtained under stirring at a temperature between -20°C and -10°C for 5-8 hours;
d) gradually raising the temperature to bring the mixture to reflux temperature and keeping it at that temperature for at least 2 hours.

10. The process according to claim 9, wherein, in said step b), the tert-butyl hydroperoxide is added to the suspension in a molar ratio with respect to the valencene variable from 2.2:1 to 2.5:1 and wherein, in said step d) the mixture is kept at reflux temperature for 2.5-4 hours.

11. The process according to any one of the preceding claims, wherein the raw nootkatone obtained from the reaction is purified by conversion into semicarbazone and subsequent acidic hydrolysis of the latter.

## Patentansprüche

1. Verfahren zur Synthese von Nootkaton aus Valencen, umfassend die Reaktion von Valencen mit einem tertiären Alkylhydroperoxid in Gegenwart von MnO₂.

2. Verfahren nach Anspruch 1, wobei das tertiäre Alkylhydroperoxid aus der Gruppe ausgewählt ist, die tert-Butylhydroperoxid, tert-Amylhydroperoxid und Di-tert-amylhydroperoxid umfasst.

3. Verfahren nach einem der Ansprüche 1 und 2, wobei die Reaktion in einem unpolaren oder einem schwach polaren Lösemittel, vorzugsweise in Dichlormethan, durchgeführt wird.

4. Verfahren nach Anspruch 3, wobei das Alkylhydroperoxid tert-Butylhydroperoxid ist.

5. Verfahren nach Anspruch 4, wobei die Reaktion bei einer Temperatur von unter oder gleich 70 °C durchgeführt wird.

6. Verfahren nach Anspruch 5, wobei die Reaktion in Dichlormethan bei einer Temperatur von unter oder gleich 40 °C, vorzugsweise bei einer Temperatur zwischen -30 °C und 40 °C, durchgeführt wird.

7. Verfahren nach einem der vorhergehenden Ansprüche, wobei das Molverhältnis zwischen tertiärem Alkylhydroperoxid und Valencen von 2 : 1 bis 3 : 1 variiert und vorzugsweise zwischen 2,2 : 1 und 2,5 : 1 beträgt.

8. Verfahren nach Anspruch 7, wobei das Mangandioxid in einem Molverhältnis, bezogen auf das Valencen, benutzt wird, das von 6 : 1 bis 12 : 1 variabel ist.

9. Verfahren nach Anspruch 4, umfassend die Schritte:
a) Herstellen einer Suspension von MnO₂ und Valencen im Molverhältnis von 6 : 1 bis 12 : 1 in Dichlormethan;
b) Hinzufügen von tert-Butylhydroperoxid zu dieser Suspension in einem Molverhältnis, bezogen auf das Valencen, das von 2 : 1 bis 3 : 1 variabel ist, bei einer Temperatur zwischen -30 °C und -10 °C;
c) Bewahren des so erhaltenen Gemisches unter Rühren für 5 bis 8 Stunden bei einer Temperatur zwischen -20 °C und -10 °C;
d) allmähliches Steigern der Temperatur, um das Gemisch auf Rückflusstemperatur zu bringen, und Halten des Gemisches auf dieser Temperatur für mindestens 2 Stunden.

10. Verfahren nach Anspruch 9, wobei in dem Schritt b) das tert-Butylhydroperoxid in einem Molverhältnis, bezogen auf das Valencen, das von 2,2 : 1 bis 2,5 : 1 variabel ist, zu der Suspension gegeben wird und wobei im Schritt d) das Gemisch für 2,5 bis 4 Stunden auf Rückflusstemperatur gehalten wird.

11. Verfahren nach einem der vorhergehenden Ansprüche, wobei das rohe Nootkaton, das aus der Reaktion gewonnen wird, durch Umwandlung zu Semicarbazon und nachfolgender saurer Hydrolyse des Letzteren gereinigt wird.

## Revendications

1. Procédé pour la synthèse de la nootkatone à partir de valencène, comprenant la réaction du valencène avec un hydroperoxyde d'alkyle tertiaire, en présence de MnO₂.

2. Procédé selon la revendication 1, dans lequel ledit hydroperoxyde d'alkyle tertiaire est sélectionné parmi le groupe comprenant l'hydroperoxyde de tert-butyle, l'hydroperoxyde de tert-amyle et l'hydroperoxyde de di-tert-amyle.

3. Procédé selon l'une quelconque des revendications 1 et 2, dans lequel ladite réaction est effectuée dans un solvant non polaire ou faiblement polaire, de préférence dans du dichlorométhane.

4. Procédé selon la revendication 3, dans lequel ledit hydroperoxyde d'alkyle est l'hydroperoxyde de tert-butyle.

5. Procédé selon la revendication 4, dans lequel ladite réaction est effectuée à une température inférieure ou égale à 70 °C.

6. Procédé selon la revendication 5, dans lequel ladite réaction est effectuée dans du dichlorométhane à une température inférieure ou égale à 40 °C, de préférence à une température entre -30 °C et 40 °C.

7. Procédé selon l'une quelconque des revendications précédentes, dans lequel le rapport molaire entre l'hydroperoxyde d'alkyle tertiaire et le valencène varie de 2:1 à 3:1 et se situe de préférence entre 2,2:1 et 2,5:1.

8. Procédé selon la revendication 7, dans lequel le rapport molaire dioxyde de manganèse/valencène est variable de 6:1 à 12:1.

9. Procédé selon la revendication 4, comprenant les étapes suivantes :
a) préparer une suspension de MnO₂ et de valencène dans un rapport molaire de 6:1 à 12:1 dans du dichlorométhane ;
b) ajouter de l'hydroperoxyde de tert-butyle à cette suspension dans un rapport molaire au valencène variable de 2:1 à 3:1, à une température comprise entre -30 °C et -10 °C ;
c) maintenir le mélange ainsi obtenu sous agitation à une température entre -20 °C et -10 °C pendant 5 à 8 heures ;
d) progressivement augmenter la température afin de porter le mélange à la température de reflux et le maintenir à cette température pendant au moins 2 heures.

10. Procédé selon la revendication 9, dans lequel, à ladite étape b), l'hydroperoxyde de tert-butyle est ajouté à la suspension dans un rapport molaire au valencène variable de 2,2:1 à 2,5:1 et dans lequel, à ladite étape d) le mélange est maintenu à la température de reflux pendant 2,5 à 4 heures.

11. Procédé selon l'une quelconque des revendications précédentes, dans lequel la nootkatone brute obtenue à partir de la réaction est purifiée par conversion en semicarbazone suivie de l'hydrolyse acide de cette dernière.
